## Europäisches Patentamt

## European Patent Office

## Office européen des brevets

(19)

(11) Veröffentlichungsnummer: **0 046 555**
**B1**

(12) **EUROPÄISCHE PATENTSCHRIFT**

(45) Veröffentlichungstag der Patentschrift:
**18.05.83**

(21) Anmeldenummer: **81106365.0**

(22) Anmeldetag: **17.08.81**

(51) Int. Cl.³: **C 07 C 17/12,** C 07 C 25/02,
C 07 B 9/00

(54) Verfahren zur Herstellung von 2,5-Dichlortoluol.

(30) Priorität: **27.08.80 DE 3032324**

(43) Veröffentlichungstag der Anmeldung:
**03.03.82 Patentblatt 82/9**

(45) Bekanntmachung des Hinweises auf die Patenterteilung:
**18.05.83 Patentblatt 83/20**

(84) Benannte Vertragsstaaten:
**CH DE FR GB LI**

(56) Entgegenhaltungen:
**FR-A-936 995**
**GB-A-503 322**
**US-A-3 000 975**

**BEILSTEINS «Handbuch der organischen Chemie»,
4. Auflage, Band 5, 1922,
VERLAG VON JULIUS SPRINGER, Berlin, Seite 296**

**Chemical Abstracts Band 72, Nr. 1, 1970 Columbus,
Ohio, USA, N.N. LEBEDEV et al. "Isomeric composition
and selectivity in the catalytic chlorination of toluene",
Seite 244, Spalte 2, abstract Nr. 2781r**

(73) Patentinhaber: **BAYER AG, Zentralbereich Patente,
Marken und Lizenzen, D-5090 Leverkusen 1, Bayerwerk
(DE)**

(72) Erfinder: **Petruck, Gerd-Michael, Dr.,
Doerpfeldstrasse 49, D-4006 Erkrath 2 (DE)**
Erfinder: **Wambach, Raimund, Dr.,
Berta-von-Suttner-Strasse 65, D-5090 Leverkusen (DE)**

ACTORUM AG

## Verfahren zur Herstellung von 2,5-Dichlortoluol

Die vorliegende Erfindung betrifft ein Verfahren zur Herstellung von 2,5-Dichlortoluol durch Chlorierung von o-Chlortoluol.

Es ist aus der US-PS 3 000 975 bekannt, Toluol in Gegenwart eines Metallchlorid-Katalysators zu chlorieren, wobei überwiegend o-Chlortoluol erhalten wird.

Weiterhin ist aus der DE-AS 25 23 104 ein Verfahren zur Herstellung von 2,5-Dichlortoluol durch Chlorierung von 2-Chlortoluol in Gegenwart von für die Kernchlorierung von Toluol üblichen Katalysatoren bekannt, das dadurch gekennzeichnet ist, dass man die Chlorierung zusätzlich in Gegenwart von Schwefel und/oder einer Schwefelverbindung, deren Schwefel mit Chlor reagieren kann und die höchstens eine Schwefel-Sauerstoff-Doppelbindung enthält, bei 0 bis 150°C durchführt.

Ausserdem ist aus der US-PS 4 031 146 ein Verfahren zur Herstellung von Dichlortoluol, das mindestens 55% 2,5-Dichlortoluol enthält, bekannt, das dadurch gekennzeichnet ist, dass o-Chlortoluol mit Chlor in Gegenwart von Metallkatalysatoren, denen als Cokatalysatoren Schwefelverbindungen zugesetzt werden, in der Weise umgesetzt wird, bis 0,5 bis 0,9 g Grammatome Chlor pro Mol o-Chlortoluol reagiert haben.

Daneben ist aus Liebigs Annalen der Chemie, 146, 321 (1868) bekannt, Dichlortoluol durch Chlorierung von Toluol in Gegenwart von Jod herzustellen.

Die oben geschilderten Verfahren haben jedoch verschiedene Nachteile. Zum einen bewirken die schwefelhaltigen Katalysatoren eine verstärkte Korrosion bei den verwendeten Reaktionsgefässen. Zum anderen sind die Schwefelverbindungen nur mit einem erheblichen Aufwand aus dem Reaktionsprodukt zu entfernen. Ausserdem stören eventuell zurückbleibender Schwefel und Schwefelverbindungen bei der Aufarbeitung des Reaktionsgemisches, da z.B. leicht flüchtige Schwefelverbindungen mit dem Reaktionsprodukt überdestillieren können.

Weiterhin ist es für die Wirtschaftlichkeit der oben erwähnten Verfahren von Nachteil, wenn bei der Reaktion teure Metallkatalysatoren wie Dichloride des Titans, Thalliums, Zinns, Zirkons, und Wolframs eingesetzt werden. Die Wirtschaftlichkeit wird zusätzlich beeinträchtigt durch die Verwendung von Katalysatoren, deren Handhabung besondere sicherheitstechnische Massnahmen erforderlich machen.

Es wurde nun ein Verfahren zur Herstellung von 2,5-Dichlortoluol durch Chlorierung von o-Chlortoluol gefunden, das dadurch gekennzeichnet ist, dass man in das o-Chlortoluol bei Temperaturen von 0 bis 150°C in Gegenwart von 0,05 bis 10 Gew.-% Jod, bezogen auf eingesetztes o-Chlortoluol, so lange Chlor einleitet, bis 0,8 bis 1,9 Mol Chlor pro Mol o-Chlortoluol umgesetzt sind.

Im erfindungsgemässen Verfahren können sowohl reines o-Chlortoluol als auch Gemisch aus Chlortoluolen verwendet werden, die mehr als 50 Gew.-% o-Chlortoluol enthalten.

Das erfindungsgemässe Verfahren wird bevorzugt in der Weise durchgeführt, dass man in das vorgelegte o-Chlortoluol solange Chlor einleitet, bis 1,0 bis 1,8 Mol Chlor, besonders bevorzugt 1,1 bis 1,6 Mol Chlor ganz besonders bevorzugt 1,4 bis 1,5 Mol Chlor pro Mol o-Chlortoluol umgesetzt sind.

In das erfindungsgemässe Verfahren wird bevorzugt 0,1 bis 5 Gew.-%, besonders bevorzugt 0,5 bis 2 Gew.-% Jod, bezogen auf eingesetztes o-Chlortoluol eingesetzt.

Das erfindungsgemässe Verfahren wird bevorzugt bei 10 bis 110°C, besonders bevorzugt bei 20 bis 80°C, durchgeführt.

Das erfindungsgemässe Verfahren kann mit oder ohne Lösungsmittel durchgeführt werden. Als Lösungsmittel können die bei der Kernchlorierung üblichen Lösungsmittel eingesetzt werden, beispielsweise chlorierte Kohlenwasserstoffe wie Methylenchlorid, Tetrachlorkohlenstoff, Ethylenchlorid und/oder Tetrachlorethan. Bevorzugt wird ohne Lösungsmittel chloriert.

Das erfindungsgemässe Verfahren kann bei Normaldruck oder erhöhtem Druck, z.B. bei 1 bis 10 bar, durchgeführt werden. Vorzugsweise wird bei Normaldruck gearbeitet.

Das erfindungsgemässe Verfahren kann kontinuierlich oder diskontinuierlich, bevorzugt kontinuierlich ausgeführt werden.

Es ist selbstverständlich auch möglich, das erfindungsgemässe Verfahren in Gegenwart der für die Kernchlorierung üblichen Katalysatoren wie Eisenchlorid, Antimonchlorid, Phosphorpentachlorid, Aluminiumchlorid und/oder Borchlorid, durchzuführen.

Statt dem vorgelegten o-Chlortoluol und Jod enthaltenden Gemisch Chlor zuzugeben, ist es jedoch auch möglich zu dem vorgelegten o-Chlortoluol gleichzeitig eine Lösung von Jod und Chlor zuzugeben. Dabei kann Jod und Chlor in den oben erwähnten organischen Lösungsmitteln gelöst werden.

Eine bevorzugte Ausführungsform des erfindungsgemässen Verfahrens sei im folgenden dargelegt:

In eine Mischung aus o-Chlortoluol und Jod wird so lange Chlor eingeleitet, bis 1,0 bis 1,6 Mol Chlor pro Mol o-Chlortoluol umgesetzt sind. Anschliessend wird das Jod aus dem Reaktionsgemisch mit Wasser bzw. besonders vorteilhaft mit einer Kaliumjodid- oder Natriumsulfit-Lösung herausgewaschen. Das so erhaltene Rohprodukt wird unter Normaldruck durch fraktionierte Destillation aufgearbeitet, wobei ein an 2,5-Dichlortoluol angereichertes Gemisch erhalten wird.

Nach dem erfindungsgemässen Verfahren wird ein Chlortoluol-Gemisch enthalten, das einen besonders hohen Anteil an 2,5-Dichlortoluol (bis über 80%) enthält.

Dies ist umso überraschender, als aus Liebigs Annalen 139, 331 (1866) bekannt ist, dass bei der Chlorierung von Toluol in Gegenwart von Jod Nebenreaktionen, wie Kernjodierungen, auftreten können, die die Ausbeute an 2,5-Dichlortoluol vermindern.

2,5-Dichlortoluol ist ein Zwischenprodukt für Herbizide und Farbstoffe (vgl. US-PS 4 032 146, DE-AS 25 23 104).

Die nachfolgenden Beispiele sollen das erfindungsgemässe Verfahren verdeutlichen, ohne es jedoch auf diese zu beschränken.

Beispiel 1

In 633 g (5 Mol) o-Chlortoluol wird nach Zugabe von 12,7 g Jod bei 20°C so lange Chlor eingeleitet, bis 1,11 Mol Chlor umgesetzt sind. Das Reaktionsgemisch hat dann eine Dichte von 1,30 g/cm$^3$ (bei 20°C). Anschliessend wird das Jod mit Wasser herausgewaschen. Man erhält 812 g Rohprodukt mit einer gaschromatographisch bestimmten Zusammensetzung, bestehend aus 1,9% Orthochlortoluol; 11,9% 2,4-Dichlortoluol; 54,2% 2,5-Dichlortoluol; 7,8% 2,6-Dichlortoluol; 5,4% 2,3-Dichlortoluol; 18,6% Trichlortoluol. Bei der fraktionierten Destillation des Rohproduktes wird das Gemisch aus Dichlortoluolen erhalten, welches 73,3% 2,5-Dichlortoluol enthält.

Beispiele 2 bis 5

Beispiele 2 bis 5 werden wie Beispiel 1 durchgeführt. Abweichende Bedingungen und Ergebnisse sind in der Tabelle wiedergegeben:

Tabelle: Beispiele 2–5
Chlorierung von o-Chlortoluol in Gegenwart von Jod

| Bei-spiel | Ansatz-grösse | Jod | Chlor-umsatz | Tempe-ratur | Dichte | Aus-beute | Zusammensetzung des Rohproduktes in %[5] | | | | | | % C in der Fraktion der Dichlor-toluole |
|---|---|---|---|---|---|---|---|---|---|---|---|---|---|
| | (molA)[1] | [g] | (mol) | °C | g/cm$^3$ | g[2] | A | B | C | D | E | F | |
| 2 | 5 | 6,4 | 5,35 | 20 | 1,30[3] | 834 | 6,2 | 11,7 | 53,6 | 8,3 | 6,0 | 14,2 | 72,8 |
| 3 | 5 | 12,7 | 5,65 | 40 | 1,31[3] | 808 | 1,4 | 11,6 | 53,2 | 7,0 | 5,0 | 21,6 | 74,1 |
| 4 | 3 | 7,6 | 3,40 | 50 | 1,30[4] | 482 | 1,2 | 11,6 | 53,2 | 6,4 | 4,8 | 22,8 | 74,1 |
| 5 | 5 | 12,6 | 7,30 | 80 | 1,38[4] | 885 | – | 6,3 | 38,9 | 2,7 | 1,8 | 50,3 | 81,2 |

[1] A = o-Chlortoluol;
[2] Gewicht des chlorierten Produktes;
[3] bei 20°C;
[4] bei 50°C;
[5] B = 2,4-, C = 2,5-, D = 2,6-, E = 2,3-Dichlortoluol, F = Trichlortoluole

## Patentansprüche

1. Verfahren zur Herstellung von 2,5-Dichlortoluol durch Chlorierung von o-Chlortoluol, dadurch gekennzeichnet, dass man in das o-Chlortoluol bei Temperaturen von 0 bis 150°C in Gegenwart von 0,05 bis 10 Gew.-% Jod, bezogen auf eingesetztes o-Chlortoluol, so lange Chlor einleitet, bis 0,8 bis 1,9 Mol Chlor pro Mol eingesetztes o-Chlortoluol umgesetzt sind.

2. Verfahren nach Anspruch 1, dadurch gekennzeichnet, dass ein Gemisch aus Chlortoluolen eingesetzt wird, das mehr als 50 Gew.-% o-Chlortoluol enthält.

3. Verfahren nach Anspruch 1 und 2, dadurch gekennzeichnet, dass so lange Chlor eingeleitet wird, bis 1,1 bis 1,6 Mol Chlor pro Mol o-Chlortoluol umgesetzt sind.

4. Verfahren nach Anspruch 1 und 2, dadurch gekennzeichnet, dass so lange Chlor eingeleitet wird, bis 1,4 bis 1,5 Mol Chlor pro Mol eingesetztes o-Chlortoluol umgesetzt sind.

5. Verfahren nach Ansprüchen 1 bis 4, dadurch gekennzeichnet, dass man 0,1 bis 5 Gew.-% Jod, bezogen auf eingesetztes o-Chlortoluol, einsetzt.

6. Verfahren nach Ansprüchen 1 bis 5, dadurch gekennzeichnet, dass man 0,5 bis 2 Gew.-% Jod, bezogen auf eingesetztes o-Chlortoluol, einsetzt.

7. Verfahren nach Ansprüchen 1 bis 6, dadurch gekennzeichnet, dass man die Umsetzung bei einer Temperatur von 10 bis 110°C durchführt.

8. Verfahren nach Ansprüchen 1 bis 7, dadurch gekennzeichnet, dass man das Verfahren bei Temperaturen von 20 bis 80°C durchführt.

9. Verfahren nach Ansprüchen 1 bis 8, dadurch gekennzeichnet, dass in Methylenchlorid, Tetrachlorkohlenstoff, Ethylenchlorid und/oder Tetrachlorethan chloriert wird.

10. Verfahren nach Ansprüchen 1 bis 9, dadurch gekennzeichnet, dass in Gegenwart von Eisenchlorid, Antimonchlorid, Phosphorpentachlorid, Aluminiumchlorid und/oder Borchlorid chloriert wird.

## Claims

1. Process for the preparation of 2,5-dichlorotoluene by chlorination of o-chlorotoluene, characterised in that chlorine is passed into the o-chlorotoluene at temperatures of 0 to 150°C in the presence of 0.05 to 10% by weight of iodine, relative to the o-chlorotoluene employed, until 0.8 to 1.9 mols of chlorine have reacted per mol of o-chlorotoluene employed.

2. Process according to Claim 1, characterised in that a mixture of chlorotoluenes which contains more than 50% by weight of o-chlorotoluene is employed.

3. Process according to Claims 1 and 2, characterised in that chlorine is passed in until 1.1 to 1.6 mols of chlorine have reacted per mol of o-chlorotoluene.

4. Process according to Claims 1 and 2, characterised in that chlorine is passed in until 1.4 to 1.5 mols of chlorine have reacted per mol of o-chlorotoluene employed.

5. Process according to Claims 1 to 4, characterised in that 0.1 to 5% by weight of iodine, relative to the o-chlorotoluene employed, is used.

6. Process according to Claims 1 to 5, characterised in that 0.5 to 2% by weight of iodine, relative to the o-chlorotoluene employed, is used.

7. Process according to Claims 1 to 6, characterised in that the reaction is carried out at a temperature of 10 to 110°C.

8. Process according to Claims 1 to 7, characterised in that the process is carried out at temperatures from 20 to 80°C.

9. Process according to Claims 1 to 8, characterised in that the chlorination is carried out in methylene chloride, carbon tetrachloride, ethylene chloride and/or tetrachloroethane.

10. Process accoridng to Claims 1 to 9, characterised in that the chlorination is carried out in the presence of iron chloride, antimony chloride, phosphorous pentachloride, aluminium chloride and/or boron chloride.

## Revendications

1. Procédé de préparation du 2,5-dichlorotoluène par chloration du l'o-chlorotoluène, caractérisé en ce que l'on injecte du chlore dans l'o-chlorotoluène à des températures de 0 à– 150°C en présence de 0,05 à 10% en poids d'iode, par rapport à l'o-chlorotoluène mis en œuvre, jusqu'à ce que 0,8 à 1,9 mole de chlore ait réagi par mole d'o-chlorotoluène mis en œuvre.

2. Procédé selon la revendication 1, caractérisé en ce que l'on part d'un mélange de chlorotoluène contenant plus de 50% en poids d'o-chlorotoluène.

3. Procédé selon les revendications 1 et 2, caractérisé en ce que l'on injecte du chlore jusqu'à ce que 1,1 à 1,6 mole de chlore ait réagi par mole d'o-chlorotoluène.

4. Procédé selon les revendications 1 et 2, caractérisé en ce que l'on injecte du chlore jusqu'à ce que 1,4 à 1,5 mole de chlore ait réagi par mole d'o-chlorotoluène mis en œuvre.

5. Procédé selon les revendications 1 à 4, caractérisé en ce que l'on utilise de 0,1 à 5% en poids d'iode par rapport à l'o-chlorotoluène mis en œuvre.

6. Procédé selon les revendications 1 à 5, caractérisé en ce que l'on utilise de 0,5 à 2% en poids d'iode par rapport à l'o-chlorotoluène mis en œuvre.

7. Procédé selon les revendications 1 à 6, caractérisé en ce que l'on effectue la réaction à une température de 10 à 110°C.

8. Procédé selon les revendications 1 à 7, caractérisé en ce que le procédé est mis en œuvre à des températures de 20 à 80°C.

9. Procédé selon les revendications 1 à 8, caractérisé en ce que l'on chlore dans le chlorure de méthylène, le tétrachlorure de carbone, le chlorure d'éthylène et/ou le tétrachloréthane.

10. Procédé selon les revendications 1 à 9, caractérisé en ce que l'on chlore en présence de chlorure de fer, de chlorure d'antimoine, de pentachlorure de phosphore, de chlorure d'aluminium et/ou de chlorure de bore.